(19) Europäisches Patentamt
European Patent Office
Office européen des brevets

(11) **EP 4 311 527 A1**

(12) **EUROPEAN PATENT APPLICATION**

(43) Date of publication:
**31.01.2024  Bulletin 2024/05**

(21) Application number: **22187697.2**

(22) Date of filing: **29.07.2022**

(51) International Patent Classification (IPC):
**A61F 2/90** (2013.01)     **D04C 1/06** (2006.01)
**D04C 3/08** (2006.01)

(52) Cooperative Patent Classification (CPC):
**B29C 53/583; D04C 1/06;** A61F 2210/0076;
D10B 2509/06

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Intressa Vascular S.A.
5032 ISNES ISNES (BE)**

(72) Inventors:
• **Doda, Kanae
5541 Hastiere-par-Dela (BE)**

• **Dreano, Loic
Lyon (FR)**
• **Lejeune, Diane
1150 Brussels (BE)**
• **Douette, Pierre
4053 Embourg (BE)**

(74) Representative: **AWA Benelux
AWA Benelux SA
Tour & Taxis - Royal Depot box:216
Havenlaan 86c Avenue du Port
1000 Bruxelles (BE)**

(54) **IMPLANTABLE 3D BRAIDED ENDOVASCULAR PROSTHESIS FOR REMODELING OF DISSECTED AORTA**

(57)    The invention relates to endovascular prostheses (1) for use in the treatment of aortic dissection, comprising a self-expandable braided framework (2). The framework (2) is formed by a plurality of layers of wires. The plurality of layers of wires (3) consists of a lumen comprised in the framework (2), forming a cylindrical shape. A ratio (T2/D3) of a thickness (T2) of the framework wall to the diameter (D3) of the wires (3) is at least 2.8. Each wire (3) forms a helical path. The number of wires belonging to the outermost layer of the framework is at least 5% and at most 35% of the total number of wires (3). The number of wires belonging to the innermost layer of the framework is at least 10% and at most 35% of the total number wires (3) forming of the self-expandable braided framework (2).

*Fig. 5*

**Description**

**Field of the invention**

The present invention relates to implantable endovascular prostheses for treatment of aortic dissection.

**Background of the invention**

The aorta is the largest blood vessel in the body and responsible for transporting oxygen rich blood from the heart to the rest of body. It is shaped like a candy cane, with the first section moving up towards the head (ascending aorta), then curving in a C-shape as smaller arteries that stem therefrom carry blood to the head and arms (aortic arch). After the curve, the aorta becomes straight again, and moves downward towards the abdomen, carrying blood to the lower part of the body (descending aorta). The walls of the aorta consist of three layers that ensure structural support and morphology.

Aortic dissection is one of the aortic catastrophes with a high mortality and morbidity. It typically occurs when the inner layer of the artery's wall weakens and a primary tear occurs into the middle layer of the wall. When this happens, blood can pass through the tear, causing the layers to separate from one another, or dissect. If left untreated, the tear can enlarge. This leads to the formation of a new channel, called a false lumen (FL), separate from the true lumen (TL) by an intimal flap. This FL can progressively extend from the tear to the lowest part of the aorta, preventing blood from flowing properly in the TL to irrigate end-organs branched onto the aorta.

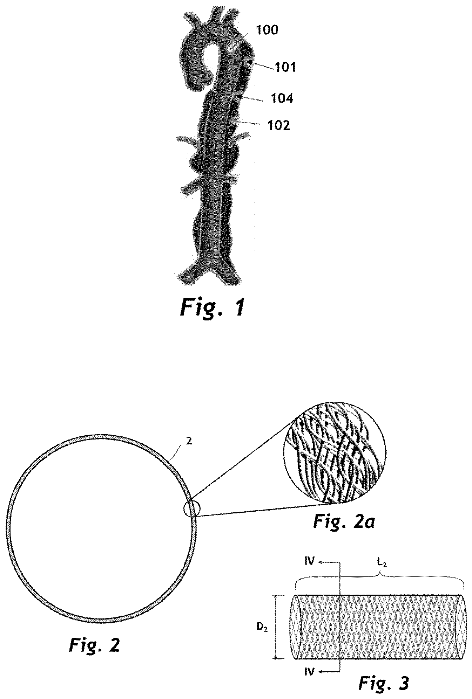

As shown in FIG.1, the separation of the inner layer of the aorta which forms the intimal flap **104** can have multiple entry holes, known as reentry tears **101.** These secondary tears allow blood to flow between the TL **100** and the FL **102.** Over time, the FL will compress the true lumen, hence reduce the flow of blood going to the end-organs leading to ischemia. Sometimes, the blood breaks through the outer layer of the aorta, a complication called aortic rupture, causing a life-threatening loss of blood and drop in blood pressure that requires immediate surgery. Aortic dissections that occur in the ascending part of the aorta are called Stanford type A dissections; those in the descending aorta are Stanford type B dissections. Type B dissections are also classified based on chronicity into hyperacute, acute, subacute and chronic wherein the hyperacute phase corresponds to the 24h first hours from the onset of the symptom, the acute phase as the time between 24h and 14 days of the onset of symptoms, subacute between 2 weeks and 3 months and chronic after 3 months.

Traditionally, type B aortic dissections (TBAD) which are uncomplicated at presentation, are treated with optimal medical therapy, and lifestyle modification to minimize risk of progression. However, up to 50% of patients may suffer aorta-related complication such as FL enlargement or rupture and retrograde dissection. If imaging shows such complications, repair should be considered. Besides, evidence is accumulating that dissections presenting as complicated (defined as rupture or with clinical signs of malperfusion) or high-risk (defined as associated with refractory pain, refractory hypertension, bloody pleural effusion, aortic diameter >40 mm, radiographic only malperfusion, readmission, an entry tear located in the lesser curve of the aorta, or a false lumen diameter >22 mm) should be treated with endovascular repair.

Indeed, stent graft endovascular repair is known as alternative option to conventional open surgical repair. It is expected to promote aortic remodeling with favorable FL regression and favorable TL expansion. The limitations of stent grafts are linked to their intrinsic natures and characteristics. First, due to the impermeable character of the graft material, placing a stent graft in front of side branches will occlude such branches and lead to undesired end-organ ischemia (e.g., spinal cord injury, kidney failure). Short stent grafts are therefore often used to limit such side effects, but come with the downside that they are not able to cover the whole dissected aorta. This can lead to continued retrograde FL perfusion with back-flow from secondary entry tears located distally from the stent graft and/or extension of dissection beyond the treated area, in turn leading to reintervention.

Additionally, incidents of late distal stent graft-induced new entry (SINE) and late extensions of aortic dissection are often reported involving the thoracic as well as the abdominal and iliac portions. It may be caused by high radial force and stiffness of stent-graft partially covering the dissected portion of aorta and pulsatile motion that erode the fragile intima at the distal edge of the stent graft.

In order to overcome these challenging problems, endovascular treatment strategies should not only target the occlusion of the proximal entry-tear but should focus in addition on the prevention of FL back-flow by providing extended mechanical support to the dissected aorta as to promote sufficient aortic remodeling.

Accordingly, there is a need for an implantable endovascular prosthesis having mechanical properties suitable to achieve favorable aortic remodeling, and allowing to cover long segments of the aorta with more entry points to reduce pressure and flow transmission into the FL, while maintaining the blood flow in branches of which the orifices are covered by said endovascular prosthesis at the thoracoabdominal level without significative risk of morbidity such as spinal cord ischemia.

## Summary of the invention

An object of the present invention is to provide a device implantable by endovascular approach for treating aortic dissection.

The subject of the invention is defined in the appended independent claims. Preferred embodiments are defined in the dependent claims.

A subject of the present invention is an implantable endovascular prosthesis comprising at least one self-expandable braided framework extending along an axis able to expand from a radially compressed state in a delivery configuration to a radially expanded state. Said braided framework is formed by wires of a same diameter $D_3$ and devoid of any impermeable cover layer. The braided framework comprises a plurality of layers of wires made of biocompatible material and forms a part of the wall of the implantable endovascular prosthesis. Said plurality of layers of wires consists of a lumen comprised in the braided framework. It forms a cylindrical shape with a circular cross-section. Each of said layers forms a mesh. Said meshes form a lattice with a plurality of wires of the layers. The meshes being interlocked. The wires are integrated in the mesh of at least one of the adjacent layers.

Each the wires forms a helical path in a direction or in the other around the axis of the self-expandable braided framework while crossing with wires of the opposite helical direction being positioning at either the outer or the inner side of the braided framework. A ratio $T_2/D_3$ of a thickness $T_2$ of a wall of said braided framework to the diameter $D_3$ of the wires is at least 2.8, preferably at least 3.0, most preferably at least 3.5. Wires taking position at the outer side the most often among wires of the same helical direction when crossing with wires of the opposite helical direction during a complete helical wrap are defined as wires belonging to the outermost layer of the braided framework (OM wires). Wires taking position at the inner side the most often among wires of the same helical direction when crossing with wires of the opposite helical direction during a complete helical wrap being defined as wires belonging to the innermost layer of the braided framework (IM wires). The number of OM wires is at least 5% and at most 35% of the total number of wires forming of the braided framework, the number of IM wires being at least 10% and at most 35% of the total number wires forming of the braided framework.

A pattern indicating positions of wires at crossing points is preferably repeating at least 4 times within a complete helical wrap of the wires in both helical directions, more preferably at least 8 times.

The number of wires forming said braided framework but neither being OM wire nor being IM wire is preferably at least 50% and at most 70% of the total number of wires forming the braided framework.

A ratio of the number of times that an OM wire crosses at outer side to the number of the total crosses made within its complete helical wrap is preferably at least 70% and at most 90%.

The number of wires forming the braided framework is preferably at least 72, more preferably at least 96, even more preferably at least 120 and 200, still even more preferably between 150 and 180.

A surface coverage ratio (SCR) of the braided framework in the fully expanded state is preferably comprised between 25% and 45%, more preferably between 30 and 40%.

The wires forming the braided framework have preferably a diameter $D_3$ of at least 120 $\mu$m and at most 250 $\mu$m, more preferably at least 150 $\mu$m and at most 220 $\mu$m, even more preferably at least 180 $\mu$m and at most 210 $\mu$m.

In a preferable embodiment, the cylindrical form of the braided framework has preferably a constant diameter in the fully expanded state. Preferably the braided framework has an inner diameter of at least 20 mm and at most 50 mm in the fully expanded state.

In another preferable embodiment, the braided framework has larger diameter one or both end(s) in the fully expanded state. Preferably an inner diameter of the braided framework is at least 20 mm and at most 50 mm in the fully expanded state.

## Brief description of the Figures

FIG.1 is a diagrammatic view of Type B aortic dissection.

FIG.2 is a schematic front view of an implantable endovascular prosthesis according to the present invention in a preferable embodiment.

FIG.2a is a schematic magnified view of a portion of the front view shown in FIG.2.

FIG.3 is a side view of the endovascular prosthesis shown in FIG.2.

FIG.4 is a section view of the endovascular prosthesis shown in FIG 2, 2a and 3 according to a cutting plane IV-IV

FIG.4a shows is a schematic magnified view of a portion of the cross-section shown in FIG 4.

FIG.5 is a schematic magnified view of a wall of a self-expandable braided framework according to present invention.

FIG.6 is a magnified image of a self-expandable braided framework according to present invention.

FIGs.7a to 7c are computed tomography (CT) scans before and after treated by an implantable endovascular prosthesis according to present invention.
FIGs.8a to 8c are computed 3D fusion images constructed based on CT scans.

## Detailed description of the invention

As used hereinafter, the term "implantable" refers to an ability of a medical device to be positioned at a location within a body vessel through invasive means. Implantable medical device can be configured for transient placement within a body vessel during a medical intervention (e.g., seconds, minutes, hours), or to remain in a body vessel permanently.

The terms "endovascular" prosthesis refers to a device adapted for invasive placement in a curved or straight body vessel by procedures wherein the prosthesis is advanced within and through the lumen of a body vessel from a remote location to a target site within the body vessel. In vascular procedures, a medical device can typically be introduced "endovascularly" using a catheter over a guide wire under fluoroscopic guidance. The catheters and wire guides may be introduced through conventional access sites in the vascular system.

The term "catheter" refers to a tube that is inserted into a blood vessel to access the target site. In the present description, a "catheter" will designate either a catheter per se, or a catheter with its accessories, meaning needle, guide wire, introducer sheath and other common suitable medical devices known by the man skilled in the art.

The term "permanent" refers to a medical device which may be placed in a blood vessel and will remain in the blood vessel for a long period of time (e.g. months, years) and possibly for the remainder of the patient's life.

The terms "expanded shape" or "expanded state" refer to a shape or state resulting from the selfexpanding properties of a self-spring-back object when it is allowed to expand without any outer compression force (i.e., non-constricted state).

The implantable endovascular prosthesis **1** according to the present invention comprises at least one self-expandable braided framework **2**. The self-expandable braided framework **2** is configured to take a compressed shape having a relatively small and relatively uniform diameter when disposed within a delivery system (i.e., "in compressed state"), and to spontaneously take a deployed shape with radially expanded diameter within the delivery location such as a body lumen (i.e., "in deployed state"). The self-expandable braided framework **2** has a 3D braided structure having a plurality of interlocked layers (interlocked multilayer configuration) formed by braiding a plurality of wires. The self-expandable braided framework **2** comprises a lumen in a cylindrical form with a circular cross-section shown in FIGs.2, 2a and 3.

In a preferable embodiment, the implantable endovascular prosthesis **1** can substantially consist of the self-expandable braided framework **2**.

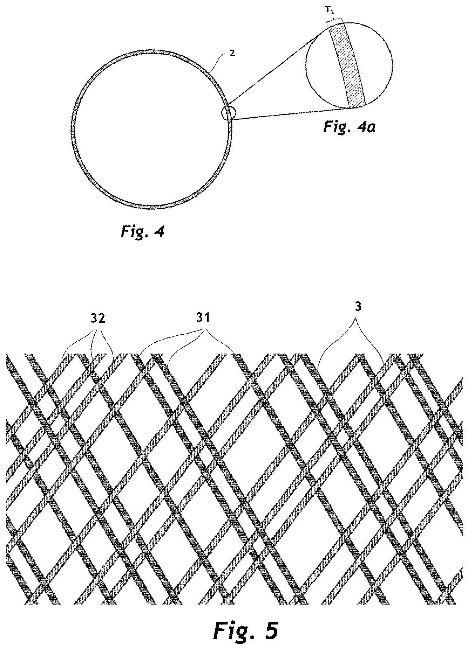

FIG.4 shows a schematic cross-section of the implantable endovascular prosthesis **1** according to the present invention. FIG.4a shows a schematic magnified view of a portion of the implantable endovascular prosthesis **1** consisting of a self-expandable framework **2**.

The self-expandable braided framework **2** has a thickness $T_2$ in a fully expanded state. It may be measured by using a Micro-CT scan technology to identify a circumscribed circle and Inscribed circle of a cross section of the braided framework **2** and to divide by 2 the difference between the diameters of these circles. The term "interlocked multilayer" refers to a framework comprising multiple layers, whose plies are not distinct at the time of braiding, for example a given number of wires of the plies of the first layer being interlocked with the plies of the second layer and/or other layers as schematically illustrated in FIG.5. Said interlocked multilayer configuration, for example, can be provided by using the braiding machine described in EP1248872. When the self-expandable braided framework **2** is observed vertically with respect to its wall, meshes of the self-expandable braided framework **2** forms lattices with a plurality of level of wires **3**.

A ratio $T_2/D_3$ of the thickness $T_2$ of a wall of the self-expandable braided framework **2** to the diameter $D_3$ of wire **3** forming of the braided framework **2** should be greater than 2.0. It characterizes that the self-expandable braided framework **2** has more than a single layer of mesh, namely 3D braided structure with a multilayered configuration. The ratio $T_2/D_3$ is preferably at least 2.8, more preferably at least 3.0, even more preferably at least 3.5. The greater the ratio $T_2/D_3$, the greater in the degree of multilayering.

The term "OM wire" refers to a wire belonging to the outermost layer of the self-expandable braided framework **2,** which can be defined as a wire taking position at the outer side the most often among wires of the same helical direction when crossing with wires of the opposite helical direction during its complete helical wrap. By most often, it is meant preferably at least 70% of the total number of crossing on a complete helical wrap. The term "IM wire" means a wire belonging to the innermost layer of the self-expandable braided framework **2,** which can be defined a wire taking position at the inner side the most often among wires of the same helical direction when crossing with wires of the opposite helical direction during its complete helical wrap. The positions of the wires at crossing point can be identified by observing vertically with respect to a wall of the self-expandable braided framework **2**. Namely, the term "position at the outer side" means a wire crossing over another wire, and the term "position at the inner side" meaning a wire crossing down another wire when a crossing point was observed vertically with respect to the wall. In a preferable embodiment, the number of OM wires included in the self-expandable braided framework **2** is at least 5% and at most 35% in the total number of wires **3** forming of the braided framework **2**; and the number of IM wires being at least 10% and at most 35% in the total number wires **3**. The number of times that "OM wire" is positioned at outer side at crosses during its helical wrap is preferably at most 90%, more preferably at least 80%. The number of times that "IM wire" is positioned at outer side at crosses during its helical wrap is preferably at least 5% and at most 20%, more preferably at least 10%. A pattern indicating such positions of wires at crossing points is preferably repeating at least 4 times within a complete helical wrap of the wires in both helical directions, more preferably at least 8 times. Wires not belonging to the outmost layer nor the innermost layer can be defined as wires belong to middle layers. The number of wires belonging to middle layers is preferably at least 50% and at most 70% in the total number of wires forming the braided framework. The more the number of times OM wire positioning at outer side, the more the implantable endovascular prosthesis being flexible while maintaining the same radial force for progressive remodeling of dissected aorta.

The surface coverage ratio (SCR) of self-expandable braided framework **2** is preferably between 25% and 45%, more preferably between 30% and 40% in fully expanded state. The SCR of the endoluminal prosthesis is defined by the relation:

$$SCR = Sw/St$$

Wherein "Sw" is the actual surface covered by wires **3** composed in the braided framework **2,** and "St" is the total surface are of the wall of the braided framework **2** when observed normal with respect to the wall.

The self-expandable braided framework **2** of the endovascular prosthesis **1** is made of at most 196 wires **3,** preferably at least 72 wires at most 160 wires. The wires preferably have a diameter $D_3$ of at least 120 $\mu$m, preferably at least 150 $\mu$m, more preferably at least 180 $\mu$m, even more at least 200 $\mu$m and at most 220 $\mu$m.

The biocompatible material used in the invention is preferably a metallic substrate selected from a group consisting of stainless steels (e.g., 316, 316L or 304); nickel-titanium alloys including shape memory or superelastic types (e.g., nitinol, Nitinol-DFT®-Platinum); cobalt-chrome alloys (e.g., elgiloy); cobalt-chromium-nickel alloys (e.g., phynox); alloys of cobalt, nickel, chromium and molybdenum (e.g., MP35N or MP20N); cobalt-chromium-vanadium alloys; cobalt-chromium-tungsten alloys; magnesium alloys; titanium alloys (e.g., TiC, TiN); tantalum alloys (e.g., TaC, TaN); L605. Said metallic substrate is preferably selected from the group consisting of titanium, nickel-titanium alloys such as nitinol and Nitinol-DFT®-Platinum, any type of stainless steels, or a cobalt-chromium-nickel alloys such as Phynox®.

One of the technical effects provided by the 3D braided structure of self-expandable braided framework **2** is that, the implantable endovascular prosthesis **1** exhibits the attribute of spontaneously deploying within TL along the curve of aorta without balloon molding, and of pushing a dissection flap so that TL will be enlarged with a concomitant decrease of the FL. Accordingly, the implantable endovascular prosthesis **1** has sufficient flexibility to conform with the curves of aorta as well as an adequate mechanical property for pushing back the collapsed wall of TL and repositioning it progressively to its original non-dissected diameter.

In addition, thanks to the permeable property of the 3D braided structure of the self-expandable braided framework **2,** the endovascular prosthesis **1** can keep the branches and collaterals unobstructed without additional repairs such as open debranching-bypass procedure and custommade fenestrated/branched configuration for maintaining a blood flow are not required.

**Examples (3D braided configuration)**

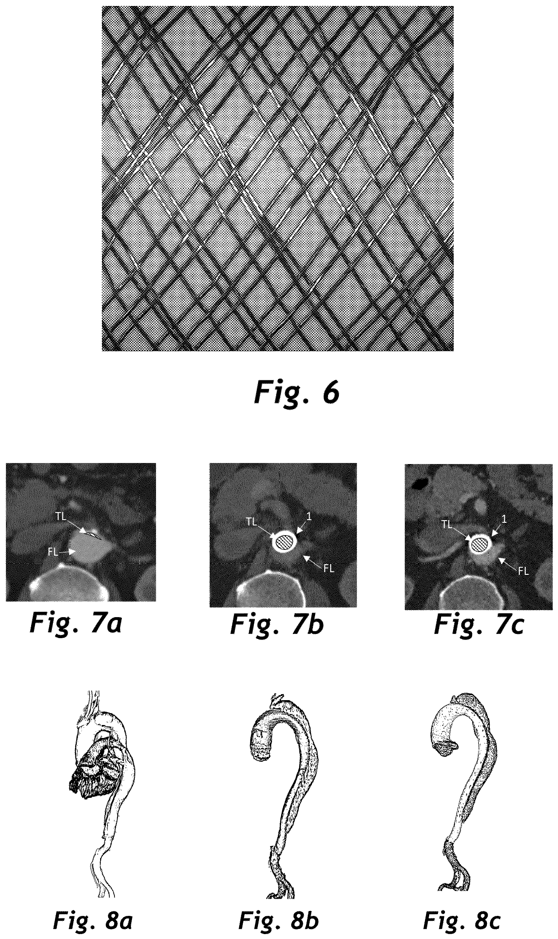

FIG.6 shows a part of a self-expandable braided framework of Example 1 (Ex.1) according to the present invention. The braiding pattern of Ex.1 was projected to Table 1. The table shows which wires were positioned at outer side of the self-expandable braided frame at each crossing point within a repeating unit. For example, when a wire helically rotating in clockwise (C01) was positioning at outer side on a crossing point with a wire in anticlockwise (A01), a letter "C" was written in a cell of C01-A01. Ex.1 was formed by 104 wires including 52 wires braided in clockwise (C-wires) and 52 wires in anticlockwise (A-wires). In a complete helical wrap (pitch), a C-wire crossed 52 times with A-wires.

Every 13 crossings, the patterns of wire positions were repeated. Therefore, the repeating unit consisted of 13 C-wires and 13 A-wires. It was repeated 4 times within the complete helical wrap. In the repeating unit, 4 out of 13 C-wires (C02, C05, C08, C11) and 2 out of 13 A-wires (A03, A09) were identified as OM wires in the repeating unit (23.1%). Therefore, in total 24 out of 104 wires forming the braided framework were identified as OM wires as these wires take position at outer side the most often (11 times out of 13) in a pitch among wires of the same helical direction as summarized in Table 2. The OM wires of both clockwise and anticlockwise rotations were positioned at outer side 11 times out of 13 crossing points (84.6%) within the repeating unit. The IM wires of both rotations were positioned twice at outer side out of 13 crossing points (15.4%) as shown in Table 1. These numbers counted in a complete helical wrap (pitch) are summarized in Table 3. The number of times that the repeating unite was found in a complete helical wrap is summarized in Table 4.

Braiding and technical properties of another examples (Ex.2) regarding self-expanded braided frameworks according to the present inventions were summarized in Tables 2 to 4 as well

**Table 1 - Repeating unit of braiding pattern of Ex.1 according to the present invention showing wires positioning at outer side where crossing with wires in the opposite helical direction.**

| | | Wires in clockwise rotation (C-wires) | | | | | | | | | | | | | Number of times that A-wires cross at outer side in a repeating unit | | |
| | | C01 | C02 | C03 | C04 | C 05 | C06 | C07 | C08 | C09 | C10 | C11 | C12 | C13 | | | |
| Wires in anticlockwise rotation (A-wires) | A01 | C | C | A | C | C | C | C | C | A | C | C | C | C | 2 | 15.4% | IM |
| | A02 | C | C | A | A | C | A | C | C | A | A | C | C | A | 6 | 46.2% | |
| | A03 | A | C | A | A | A | A | A | C | A | A | A | A | A | 11 | 84.6% | OM |
| | A04 | C | C | C | C | C | A | C | C | C | C | C | C | A | 2 | 15.4% | IM |
| | A05 | A | C | A | C | C | A | A | C | A | C | C | C | A | 6 | 46.2% | |
| | A06 | A | A | A | A | C | A | A | A | A | A | C | C | A | 10 | 76.9% | |
| | A07 | C | C | A | C | C | C | C | C | A | C | C | C | C | 2 | 15.4% | IM |
| | A08 | C | C | A | A | C | A | C | C | A | A | C | C | A | 6 | 46.2% | |
| | A09 | A | C | A | A | A | A | A | C | A | A | A | A | A | 11 | 84.6% | OM |
| | A10 | C | C | C | C | C | A | C | C | C | C | C | A | A | 3 | 23.1% | |
| | A11 | C | C | C | C | C | A | C | C | C | C | C | C | A | 2 | 15.4% | IM |
| | A12 | A | C | A | C | C | A | A | C | A | C | C | C | A | 6 | 46.2% | |
| | A13 | A | A | A | A | C | A | A | A | A | A | C | C | A | 10 | 76.9% | |
| Number of times that C-wires cross at outer side in a repeating unit | | 7 | 11 | 3 | 7 | 11 | 2 | 7 | 11 | 3 | 7 | 11 | 10 | 2 | | | |
| | | 53.8% | 84.6% | 23.1% | 53.8% | 84.6% | 15.4% | 53.8% | 84.6% | 23.1% | 53.8% | 84.6% | 76.9% | 15.4% | | | |
| | | | OM | | | OM | IM | | OM | | | OM | | IM | | | |

**Table 2** - **The number of wires belonging to outmost layer, innermost layer or middle layers in each helical direction and its total numbers**

| | Number of wires | Number of wires defined as OM wires (%) | Number of wires defined as IM wires (%) | Number of wires belonging to middle layers (%) |
| --- | --- | --- | --- | --- |
| Ex. 1 | 104 | 24 (23.1%) | 24 (23.1%) | 56 (53.8%) |
| Ex. 2 | 112 | 24 (21.4%) | 20 (17.9%) | 68 (60.7%) |

**Table 3 - The number of times that a wire crosses at outer side with wires helically braided in the opposite direction within a complete helical wrap**

|  | Total number of crossing points per wire within a complete helical wrap | Wire type | | |
|---|---|---|---|---|
|  |  | OM wire | IM wire | Wires belonging to middle layers |
| Ex. 1 | 52 | 44 (84.6%) | 8 (15.4%) | 12-40 (23-77%) |
| Ex. 2 | 56 | 48 (85.7%) | 4 (7.1%) | 16-40 (29-71%) |

**Table 4 - Number of times that a repeating unit is found within a complete helical wrap**

|  | Number of wires | Number of wires /repeating unit /helical direction | Number of repeating units/ pitch |
|---|---|---|---|
| Ex. 1 | 104 | 13 | 4 |
| Ex. 2 | 112 | 14 | 4 |

Clinical results:

A case of complicated type B dissection was treated by implanting endovascular prostheses according to the present invention from the ascending aorta to the abdominal aorta. FIGs.7a to 7c show computed tomography (CT) scans of this case sliced at the plane of maximum compression where maximum compression of the TL was observed preoperatively. At preoperative stage, the TL was collapsed by compression from the FL while branch perfusion (Celiac Trunk and left renal artery) was originating from patent aortic false lumen (FIGs.7a and 8a). During an implanting procedure, the endovascular prostheses according to the present invention was deployed along the curve of aortic arch from the ascending aorta to the abdominal aorta into the TL while covering an entry tear and a reentry tear of FL as well as orifices of aortic branches including the brachiocephalic trunk, common carotid arteries, subclavian arteries, lumbar arteries, Celiac trunk, renal arteries. At postoperative evaluation, TL was increased through re-approximation of the intimal flap after deployment of the endovascular prosthesis with a concomitant decrease of the FL (FIG. 8b). At 36-month Follow-up, the endovascular prothesis provided support to the TL resulting in progressive decrease of the size of FL while maintaining the blood flow into the branches (FIG.7c).

In conclusion, the implantable endovascular prosthesis according to the present invention exhibited sufficient flexibility to conform with the curves of aorta as well as adequate mechanical properties for allowing re-approximation and support of the intimal flap, re-expanding the TL diameter and restoring blood flow into the TL. As a result, the endovascular prosthesis led to a successful remodeling of dissected aorta while keeping appropriate blood circulation into the aortic branches even when the orifices of those branches were covered by the prosthesis.

## Claims

1. Implantable endovascular prosthesis (1) for use in the treatment of aortic dissection comprising at least one self-expandable braided framework (2) extending along an axis able to expand from a radially compressed state in a delivery configuration to a radially expanded state; the self-expandable braided framework (2) being formed by wires (3) of a same diameter ($D_3$), devoid of any impermeable cover layer, comprising a plurality of layers of wires (3) made of biocompatible material, and forming a part of the wall of the implantable endovascular prosthesis (1); the plurality of layers of wires (3) consisting of a lumen comprised in the self-expandable braided framework (2), forming a cylindrical shape with a circular cross-section; each layer forming a mesh; the meshes forming a lattice with a plurality of wires of said layers; the meshes being interlocked; the wires (3) being integrated in the mesh of at least one of the adjacent layers; each wire (3) forming a helical path in a direction or in the other around the axis of the self-expandable braided framework (2) while crossing with wires of the opposite helical direction being positioned at either the outer or the inner side of the self-expandable braided framework (2); wherein a ratio ($T_2/D_3$) of a thickness ($T_2$) of a wall of said self-expandable braided framework (2) to the diameter ($D_3$) of the wires (3) is at least 2.8, preferably at least 3.0, most preferably at least 3.5,

wherein wires taking position at the outer side the most often among wires of the same helical direction when crossing with wires of the opposite helical direction during a complete helical wrap are defined as wires belonging to the outermost layer of the self-expandable braided framework (OM wires); wires taking position at the outer side the least often among wires of the same helical direction when crossing with wires of the opposite helical direction during a complete helical wrap being defined as wires belonging to the innermost layer of the self-expandable braided framework (IM wires); wires not being OM wire nor IM wire being defined as wires belonging to middle layers of the self-expandable framework,
**characterized in that** the number of OM wires is at least 5% and at most 35% of the total number of wires (3) forming of the self-expandable braided framework (2), the number of IM wires being at least 10% and at most 35% of the total number wires (3) forming of the self-expandable braided framework (2).

2. The implantable endovascular prosthesis (1) according to claim 1 wherein a pattern indicating positions of wires at crossing points is repeated at least 4 times within a complete helical wrap of the wires in both helical directions, preferably at least 8 times.

3. The implantable endovascular prosthesis (1) according claim 1 or 2 wherein the number of wires not being OM wire nor IM wire is at least 50% and at most 70% to the total number of wires forming the self-expandable braided framework (2).

4. The implantable endovascular prosthesis (1) according to any one of the preceding claims wherein a ratio of the number of times that an OM wire crosses at outer side to the number of the total crosses made within its complete helical wrap is at least 70% and at most 90%.

5. The implantable endovascular prosthesis (1) according to any one of the preceding claims wherein the number of wires forming the self-expandable braided framework is at least 72, preferably at least 96, more preferably at least 120 and 200, even more preferably between 150 and 180.

6. The implantable endovascular prosthesis (1) according any one of the preceding claims wherein a surface coverage ratio (SCR) of the self-expandable braided framework (2) in the fully expanded state is comprised between 25% and 45%, preferably between 30 and 40%.

7. The implantable endovascular prosthesis (1) according any one of the preceding claims wherein the wires (3) forming the self-expandable braided framework (2) have a diameter ($D_3$) of at least 120 $\mu$m and at most 250 $\mu$m, preferably at least 150 $\mu$m and at most 220 $\mu$m, more preferably at least 180 $\mu$m and at most 210 $\mu$m.

8. The implantable endovascular prosthesis (1) according any one of the preceding claims wherein the cylindrical form of the self-expandable braided framework (2) has a constant diameter in the fully expanded state.

9. The implantable endovascular prosthesis (1) according to claim 8 wherein the self-expandable braided framework has an inner diameter of at least 20 mm and at most 50 mm in the fully expanded state.

10. The implantable endovascular prosthesis (1) according any one of claims 1 to 7 wherein the self-expandable braided framework (2) has larger diameter one or both end(s) in the fully expanded state.

11. The implantable endovascular prosthesis (1) according to claim 10 wherein an inner diameter of the self-expandable braided framework (2) is at least 20 mm and at most 50 mm in the fully expanded state.

**Fig. 1**

**Fig. 2a**

**Fig. 2**

**Fig. 3**

Fig. 4a

Fig. 4

Fig. 5

Fig. 6

Fig. 7a  Fig. 7b  Fig. 7c

Fig. 8a  Fig. 8b  Fig. 8c

Europäisches
Patentamt

European
Patent Office

Office européen
des brevets

**EUROPEAN SEARCH REPORT**

Application Number

EP 22 18 7697

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2004/215332 A1 (FRID NOUREDDINE [BE]) 28 October 2004 (2004-10-28) * paragraphs [0001], [0013], [0020], [0031] - [0034] * * figures * | 1-11 | INV. A61F2/90 D04C1/06 D04C3/08 |
| A | US 2004/073293 A1 (THOMPSON PAUL J [US]) 15 April 2004 (2004-04-15) * paragraphs [0013], [0018], [0027], [0056] - [0059], [0092] - [0094] * * figures 5,6,15-17 * | 1-11 | |
| A | US 2018/064525 A1 (FRID NOUREDDINE [BE]) 8 March 2018 (2018-03-08) * paragraphs [0001], [0013], [0020], [0031] - [0034] * * figures 1,12,13,15-19 * | 1-11 | |
| A | US 2015/297374 A1 (SLAZAS ROBERT [US] ET AL) 22 October 2015 (2015-10-22) * paragraph [0024] * * figures 1,12,13,15-19 * | 1-11 | TECHNICAL FIELDS SEARCHED (IPC) |
| A | US 2016/374841 A1 (SIMPSON CHARLES L [US]) 29 December 2016 (2016-12-29) * paragraphs [0067], [0077], [0099]; figures 4A,9A,18 * | 1-11 | A61F D04C |

The present search report has been drawn up for all claims

| Place of search | Date of completion of the search | Examiner |
|---|---|---|
| The Hague | 21 December 2022 | Paquay, Jeannot |

EPO FORM 1503 03.82 (P04C01)

## ANNEX TO THE EUROPEAN SEARCH REPORT
## ON EUROPEAN PATENT APPLICATION NO.

EP 22 18 7697

21-12-2022

| Patent document cited in search report | | Publication date | Patent family member(s) | | Publication date |
|---|---|---|---|---|---|
| US 2004215332 | A1 | 28-10-2004 | AT | 362735 T | 15-06-2007 |
| | | | AU | 2467002 A | 24-06-2002 |
| | | | BE | 1013757 A6 | 02-07-2002 |
| | | | CN | 1479597 A | 03-03-2004 |
| | | | DE | 60128588 T2 | 07-02-2008 |
| | | | DK | 1357857 T3 | 24-09-2007 |
| | | | EP | 1214917 A1 | 19-06-2002 |
| | | | EP | 1357857 A1 | 05-11-2003 |
| | | | ES | 2289012 T3 | 01-02-2008 |
| | | | JP | 4618978 B2 | 26-01-2011 |
| | | | JP | 2004520101 A | 08-07-2004 |
| | | | PT | 1357857 E | 02-10-2007 |
| | | | US | 2004215332 A1 | 28-10-2004 |
| | | | WO | 0247579 A1 | 20-06-2002 |
| US 2004073293 | A1 | 15-04-2004 | US | 2001056299 A1 | 27-12-2001 |
| | | | US | 2004073293 A1 | 15-04-2004 |
| US 2018064525 | A1 | 08-03-2018 | AU | 2016245170 A1 | 12-10-2017 |
| | | | BR | 112017021398 A2 | 03-07-2018 |
| | | | CA | 2980828 A1 | 13-10-2016 |
| | | | CN | 107530177 A | 02-01-2018 |
| | | | EP | 3078350 A1 | 12-10-2016 |
| | | | JP | 2018515175 A | 14-06-2018 |
| | | | KR | 20170135917 A | 08-12-2017 |
| | | | RU | 2017134464 A | 13-05-2019 |
| | | | US | 2018064525 A1 | 08-03-2018 |
| | | | WO | 2016162402 A1 | 13-10-2016 |
| US 2015297374 | A1 | 22-10-2015 | AU | 2014201192 A1 | 02-10-2014 |
| | | | AU | 2018226374 A1 | 27-09-2018 |
| | | | BR | 102014005968 A2 | 06-10-2015 |
| | | | CA | 2843912 A1 | 13-09-2014 |
| | | | CN | 104042297 A | 17-09-2014 |
| | | | DK | 2777641 T3 | 02-10-2017 |
| | | | EP | 2777641 A1 | 17-09-2014 |
| | | | JP | 6444602 B2 | 26-12-2018 |
| | | | JP | 2014176656 A | 25-09-2014 |
| | | | KR | 20140112403 A | 23-09-2014 |
| | | | US | 2014277386 A1 | 18-09-2014 |
| | | | US | 2015297374 A1 | 22-10-2015 |
| US 2016374841 | A1 | 29-12-2016 | CA | 2663961 A1 | 29-05-2008 |
| | | | EP | 2073885 A2 | 01-07-2009 |
| | | | EP | 2462975 A1 | 13-06-2012 |
| | | | EP | 2711045 A2 | 26-03-2014 |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

EP 22 18 7697

This annex lists the patent family members relating to the patent documents cited in the above-mentioned European search report.
The members are as contained in the European Patent Office EDP file on
The European Patent Office is in no way liable for these particulars which are merely given for the purpose of information.

21-12-2022

| Patent document cited in search report | Publication date | Patent family member(s) | | Publication date |
|---|---|---|---|---|
| | | EP | 3384953 A1 | 10-10-2018 |
| | | ES | 2764436 T3 | 03-06-2020 |
| | | JP | 5337037 B2 | 06-11-2013 |
| | | JP | 5820431 B2 | 24-11-2015 |
| | | JP | 6247266 B2 | 13-12-2017 |
| | | JP | 2010514462 A | 06-05-2010 |
| | | JP | 2013236935 A | 28-11-2013 |
| | | JP | 2016041254 A | 31-03-2016 |
| | | US | 2010023047 A1 | 28-01-2010 |
| | | US | 2016374841 A1 | 29-12-2016 |
| | | US | 2019231570 A1 | 01-08-2019 |
| | | US | 2021298932 A1 | 30-09-2021 |
| | | WO | 2008063782 A2 | 29-05-2008 |

EPO FORM P0459

For more details about this annex : see Official Journal of the European Patent Office, No. 12/82

**EP 4 311 527 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1248872 A

**15**